# EUROPEAN PATENT SPECIFICATION

(11) **EP 4 410 358 B1**
(45) Date of publication and mention of the grant of the patent: **06.08.2025**
(21) Application number: 23154859.5
(22) Date of filing: 03.02.2023
(51) Int. Cl.: A61N 1/05, H01R 24/00, H01R 43/00

(54) **METHOD FOR MANUFACTURING AN IMPLANTABLE LEAD AND IMPLANTABLE LEAD**
VERFAHREN ZUR HERSTELLUNG EINER IMPLANTIERBAREN LEITUNG UND IMPLANTIERBARE LEITUNG
PROCÉDÉ DE FABRICATION D'UN FIL IMPLANTABLE ET FIL IMPLANTABLE

(43) Date of publication of application: 07.08.2024
(73) Proprietor: BIOTRONIK SE & Co. KG, 12359 Berlin (DE)
(72) Inventor: Buchner, Dagmar, 10245 Berlin (DE); Thürkow, Andreas, 12107 Berlin (DE); Feldmann, Jörg, 12683 Berlin (DE)
(74) Representative: Biotronik Corporate Services SE

(56) References cited:
- EP-B1- 0 430 837
- DE-A1- 102005 039 038
- DE-B4- 102019 218 477
- US-A- 5 251 643
- US-A1- 2014 303 702
- US-B2- 8 554 299
- US-B2- 9 533 119

## Description

The present invention relates to a method for manufacturing an implantable lead and to an implantable lead as respectively defined in appended claims 1 and 9.

Implantable leads comprising an elongated lead body with ring electrodes can be used in the context of implantable medical devices such as neurostimulators for pain management.

Usually, a ring electrode is electrically and mechanically connected to a conductor of a lead body by welding. Documents DE 10 2005 039 038 A1 and EP 2 789 367 A1 disclose examples of implantable leads comprising ring electrodes with a combination of an inner and an outer sleeve, wherein the conductor is fixed between and electrically contacted by the inner and the outer sleeve. However, such clamping sleeves have the disadvantage that material of the conductor (e.g. silver or silver alloy) may leak from the sleeves and thereby enter the patient's body after implantation.

Leaking of conducting material from conductors was already prevented by sealing with silicone adhesive which material was applied to affected places of conductors. However, such adhesive application is a complex procedure and needs a corresponding space condition. Additionally, a silicone adhesive does not sufficiently seal a lead body comprising an electrically isolating layer of polyurethane composition, for example polycarbonate-based thermoplastic polyurethane (Carbothane^{®}).

Further, the disclosure of document US 9,533,119 B2 relates, generally, to an electrical lead and, more particularly, to a method of fabricating an electrical lead and to an electrical lead. The electrical lead is particularly suitable for use as a catheter sheath of a catheter assembly. The method comprises providing an elongate multi-lumen tubular member of a nonconductive material, the tubular member defining a plurality of lumens including a conductor lumen; accessing the conductor lumen externally of the tubular member by making a longitudinally extending incision in a wall of the tubular member to form at least one aperture in the wall of the tubular member proximate a distal end of the tubular member and to access the conductor lumen; inserting a plurality of conductors into the conductor lumen through the incision and feeding the plurality of conductors toward a proximal end of the tubular member so that a distal end of each conductor protrudes from the incision; attaching at least one electrically conductive element to an exposed distal end of at least one conductor of the plurality of conductors; mounting the at least one electrically conductive element on an external surface of the tubular member; and treating the tubular member to close off the at least one aperture.

The present invention is directed toward a cost-effective manufacturing method creating non-leaking implantable leads and a corresponding non-leaking implantable lead.

The above object of the present invention is achieved by a method for manufacturing an implantable lead with the features of claim 1 and an implantable lead with the features of claim 9.

In particular, the object is achieved by a method for manufacturing an implantable lead comprising an elongated lead body with a plurality of wires or wire bundles and an electrically isolating outer layer with the following steps:
- Providing sleeve having a sealed end and an open cavity opposite the sealed end and comprising electrically conducting material;
- Pulling at least one wire or wire bundle out of the lead body;
- Positioning the pulled end of the at least one wire or wire bundle within the cavity of the sleeve;
- Forming the sleeve using a pressing tool such that the volume of the cavity is reduced, that the wire or wire bundle is fixed within the sleeve and that an electrical connection of an electrical conducting material of an electrical conducting material of the at least one wire or wire bundle and the formed sleeve is established;
- Attaching a contact element to the lead body such that the contact element fixes the formed sleeve at the lead body and establishes an electrical connection to the sleeve.

The implantable lead may be a surgical lead, may be part of an implantable neurostimulator such as, for example, a spinal cord stimulator. Such implantable lead may comprise a contact paddle at its end opposite the contact element. Accordingly, such implantable lead may be used for neurostimulation, e.g., spinal cord stimulation. Alternatively, the implantable lead may be used for cardiological stimulation, e.g. in case of bradycardia or tachycardia.

The above method may be carried out once or at least twice for one lead for different wires or wire bundles, wherein in the latter case several contact places (e.g. ring electrodes) are formed.

The sleeve may be a tubular sleeve having a hollow cylinder section or hollow cuboid section or similar hollow shape section, each forming the cavity at one end. At the opposite end along the longitudinal direction of the hollow section or cavity (i.e. the direction of the longitudinal axis), the sleeve is sealed, for example hermetically sealed. The sleeve is a comparable small element having a length in longitudinal direction of 1 mm to 10 mm, for example of 1 mm to 5 mm. The outer diameter of the sleeve at the end with the cavity may be 0,3 mm to 3 mm, for example 0,3 mm to 1 mm.

As indicated above, the sleeve comprises or may consist of electrically conducting material, for example at least one material of the group comprising platinum/iridium alloys, MP35N, titanium and implantable stainless steel.

In one embodiment of the method, the end of the sleeve is sealed by pressing and/or welding, for example laser welding or resistance welding. The pressing may be provided by pliers, crimping pliers or crimping systems such as toggle presses or servo presses with corresponding punches and dies. Alternatively or additionally, laser welding or resistance welding may be provided as an additional method for securing or further fastening.

During the above manufacturing method, at least one wire or wire bundle is pulled out of the lead body (manually) with tweezers. As a result, one end of the wire or wire bundle extends from the outer surface of the lead body. This end is positioned within the cavity of the sleeve, wherein the inner surface of the sealed end provides a stop surface for the wire end or wire bundle end. It is noted that the cavity of the sleeve (prior the pressing step) has a size such that the pulled wire or wire bundle can easily be introduced into this cavity. Accordingly, the inner diameter of the open cavity of the sleeve is greater than the outer diameter of the pulled wire or wire bundle.

After the end of the pulled wire or wire bundle is accommodated within the cavity of the sleeve, for example so far that it abuts the inner surface of the sealed end, the sleeve is formed using a pressing tool such that the volume of the cavity is reduced, that the wire or wire bundle is fixed within the sleeve and that an electrical connection of the at least one wire or wire bundle and the formed sleeve is established. For example, the cavity is flattened by pressing such that the material of the sleeve displaces any electrically isolating material of the wire or wire bundle thereby establishing the electrical connection to the conducting material of the wire or wire bundle. Further, the forming of the sleeve is provided such that the sleeve is fixed, for example permanently fixed, to the wire or wire bundle. In one embodiment, the inner surface of the open cavity may comprise a surface structure, e.g. at least one protrusion, that supports displacement of isolating material of the wire or wire bundle and/or fixation of the sleeve to the wire or wire bundle. As a result of the forming step, the sleeve (with the wire or wire bundle end) has a flattened form and firmly, securely and sealingly surrounds the wire or wire bundle end accommodated within the sleeve.

After forming of the sleeve, a contact element is attached to the sleeve such that the contact element fixes the formed sleeve at the lead body (e.g. the outer surface of the lead body) by a frictional connection and establishes an electrical connection to the sleeve. The contact element may, for example, be an electrode or a connector. The contact element may be fixed or attached to the lead body by a frictional connection and/or by a positive-locking connection.

The above method provides an implantable lead which does not leak any internal material of the wire or wire bundle because it is securely sealed by the sleeve having a sealed end. This applies to leads comprising a lead body with a silicone outer layer as well as other electrically isolating materials such as thermoplastic polyurethane. Additionally, the method is simple, cost effective and provides at the same time a good mechanical fixation as well as reliable electrical connection to the contact element. Furthermore, the method is automatable. Furthermore, no separate assembly (beside the simple sleeve) is necessary for one point of contact (e.g. one ring electrode). Welding, fixation by a glue or any other additional sealing step is not needed.

In one embodiment of the method, the contact element is an annular electrode, e.g. a ring-like electrode, wherein the formed sleeve is fixed between the annular electrode and said outer layer of the lead body. The lead body is introduced into the annular electrode and the electrode is moved along the longitudinal direction of the lead body until it reaches the sleeve. The annular electrode is moved over the sleeve such that the formed sleeve is fixed as indicated above. The annular electrode is formed as a ring with an inner diameter that is little greater than the outer diameter of the lead body so that the sleeve is clamped between the outer surface of the elongated body and the annular electrode thereby forming a frictional connection. The annular electrode and the sleeve are thereby fixed at the elongated body.

In one embodiment of the method, an annular element is provided and positioned at the outer layer such that the formed sleeve is located between the annular element and the annular electrode. For example, the annular element is a ring-shaped element with an inner diameter that is approximately the outer diameter of the lead body. It is positioned at the lead body prior attachment of the contact element to the lead body. In one embodiment, it is positioned at the lead body prior pulling the at least one wire or wire bundle out of the lead body. In one embodiment, it is positioned in proximity to the opening in the electrically isolating outer layer through which the pulled wire or wire bundle extends to the outside. Further, the inner diameter of the annular electrode is greater than the outer diameter of the annular element. The advantage of the annular element is that the space requirement is reduced and the electrode as a whole does not grow in diameter.

In one embodiment of the method, the annular electrode is accommodated such that it fully covers the formed sleeve and the length of the annular electrode in longitudinal direction (i.e. the direction of the longitudinal axis of the annular electrode) is greater than the length of the formed sleeve (measured with regard to the longitudinal direction, as well). Accordingly, the length of the annular electrode may be, for example, between 1 mm and 10 mm, e.g. between 1 mm and 5 mm.

In one embodiment of the method, the sleeve is pressed into an arcuate shape. This means that the whole sleeve (including the wire end or wire bundle end) is slightly bent during pressing so that it adapts to the shape of the outer surface of the lead body/annular electrode or, if applicable, of the annular element. The formed arc extends in a direction that is perpendicular the longitudinal direction (i.e. the direction of the longitudinal axis of the sleeve). Accordingly, the thickness of the lead body at the position of the sleeve is reduced. In one embodiment of the method, the fixing of the annular electrode may permanent or temporarily. The fixing is provided by form fitting or bending, for example, by crimping, pressing or compressing by hand or machine.

In one embodiment of the method, another electrically isolating layer is applied at the outer surface of the annular electrode. For example, the another electrically isolating comprises or consists of at least one material of the group comprising a polyurethane composition, for example polycarbonate-based thermoplastic polyurethane (Carbothane^{®}), Pellethan, PTFE, Kapton or PE, and may be applied by thermal forming (shrinking/baking in the oven), coating or bonding to the surface of the annular electrode.

The above object is further achieved by an implantable lead manufactured by the above method.

Further, the above object is achieved by an implantable lead as defined in claim 9 and comprising an elongated lead body with a plurality of wires or wire bundles and an electrically isolating outer layer, wherein at least one predetermined wire or wire bundle passes through said outer layer and is positioned within an inner cavity of a formed sleeve , wherein the implantable lead further comprises a contact element that fixes the formed sleeve at the lead body and is electrically connected to the contact element. In one embodiment, the contact element is an annular electrode which covers and fixes the formed sleeve at the outer surface of the lead body. The annular electrode is described in more detail above and below.

The above implantable lead and its embodiments described below have similar advantages as indicated with regard to the method above. Further, as indicated above, the implantable lead may comprise one contact point (e.g. ring electrode) at one opening where the at least one wire or wire bundle passes through the outer electrically isolating layer or at least two of these contact points (e.g. ring electrodes), each of which comprising the sleeve and the contact element. Several such contact points may form a connector at the lead.

The outer layer of the lead body may comprise at least one electrically isolating and biocompatible material of the group comprising thermoplastic polyurethane, ETFE and PTFE.

In one embodiment of the lead the at least one wire or wire bundle is accommodated within the lead body in a helical or meandering shape. The plurality of wires or wire bundles may form a network of wires or wire bundles within the lead body.

According to an embodiment, the wire may be a stranded wire. In other words, the wire may comprise a plurality of individual wires which may form the core of the (stranded) wire. The individual wires may be twisted and/or braided together to form an electrically conductive rope. This makes the wire more robust and more flexible.

The core of one wire or wire bundle may comprise at least one electrically conductive material of the group comprising silver, a silver alloy and MP35N. The wire bundle is, for example, a rope comprising a plurality of strands (a wire having a core with multiple strands may also be called a stranded wire). Each wire or wire bundle may comprise a sheathing comprising at least one electrically isolating material of the group comprising a thermoplastic polyurethane, ETFE or PTFE.

In one embodiment of the lead the annular electrode covers the at least one wire or wire bundle extending between the formed sleeve and an opening in the outer layer through which said wire or wire bundle passes said outer layer. Accordingly, the annular electrode not only covers the formed sleeve but also the wire or wire strand extending between the opening and the sleeve. Hence, the wire or wire strand is fully covered and thereby prevented from leakage not only by the sleeve but also by this annular electrode. Accordingly, in one embodiment the length of the annular electrode is greater than the length of the formed sleeve.

The annular electrode may comprise or consist of at least one biocompatible and electrically conductive material of the group comprising stainless steel, nickel alloy, e.g., MP35N, platinum, titanium or a metal comprising a platinum compound, e.g., platinum-iridium. The annular electrode may be formed as a closed ring that is non-slotted.

In one embodiment the lead further comprises an annular element, wherein the annular element is located at the outer layer and the formed sleeve is located between the annular element and the annular electrode, wherein the length of the annular electrode is greater than the length of the annular element. The annular element may comprise or consist of the same material as the annular electrode or at least one different material, for example, biocompatible metals.

In one embodiment of the lead the annular electrode comprises the another electrically isolating layer at its outer surface as described above.

The present invention will now be described in further detail with reference to the accompanying schematic drawings, wherein
- Fig. 1: shows an embodiment of an implantable lead in a side view;
- Fig. 2: depicts a section of a second embodiment of an implantable lead during a manufacturing step prior forming by pressing in a longitudinal section;
- Fig. 3: shows the sleeve and an end section of a wire bundle of the embodiment of Fig. 2 in a longitudinal section prior the pressing step;
- Fig. 4: a section of the embodiment Fig. 2 after the last manufacturing step in a longitudinal section;
- Fig. 5: a section of a third embodiment of an implantable lead during a manufacturing step prior forming by pressing in a longitudinal section;
- Fig. 6: a section of the embodiment Fig. 5 after the last manufacturing step in a longitudinal section; and
- Fig. 7: the sleeve of the third embodiment of an implantable lead after forming by pressing in a top view.

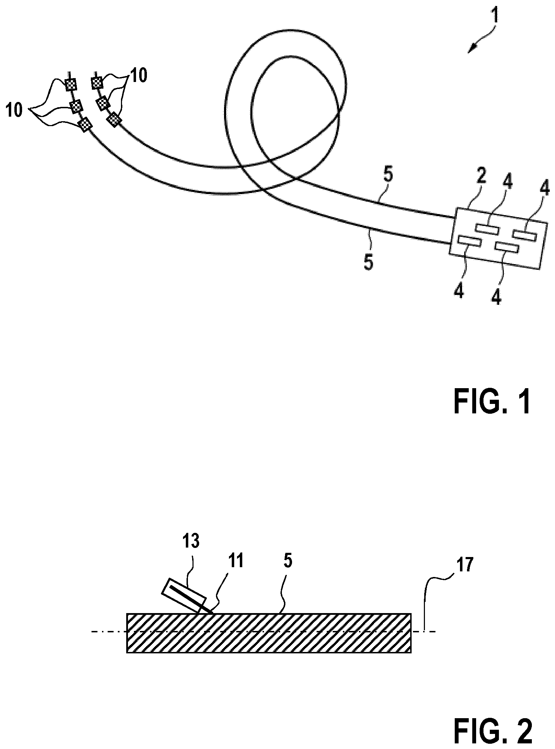

Fig. 1 shows an implantable lead 1 which may be part of an implantable neurostimulator such as, for example, a spinal cord stimulator. In this example, the implantable lead 1 comprises a contact paddle 2 on which a plurality of contact elements 4 for contacting body tissue, e.g., a portion of a spinal cord, are arranged in a specific pattern. The contact paddle 2 may have two electrical poles. Each of the poles may be connected via an elongated lead body 5, which may be made of a biocompatible and electrically insulating material, e.g., in the form of a polyurethane tube, to ring electrodes 10, wherein three ring electrodes form one connector a proximal end of the implantable lead 1. The contact paddle 2 may be attached to the lead bodies 5 at a distal end of the implantable lead 1. Each connector may be connectable to a port of an implantable pulse generator of the neurostimulator.

The manufacturing procedure of one ring electrode 10 for such lead embodiment or a similar lead is explained with reference to Fig. 2 to 4 in the following.

In the first step, a lead body 5 and a sleeve 13 is provided, wherein the lead body 5 comprises a plurality of wires or wire bundles wherein each wire or wire bundle extends along the longitudinal direction of the lead body in a net-like or helical shape. One wire bundle may be realized as a wire strand which may have a core comprising silver or a silver alloy. Further, the lead body comprises an electrically isolating outer layer, for example, of thermoplastic and biocompatible polyurethane.

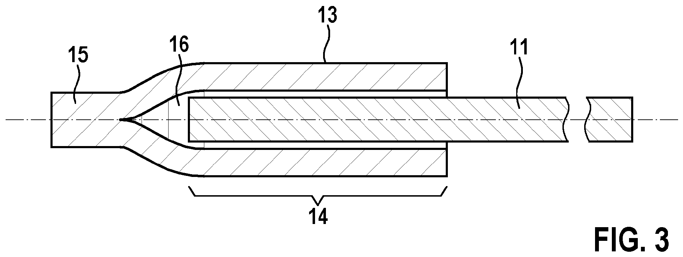

The sleeve 13 which is shown in more detail in Fig. 3 comprises a tubular section 14 forming a cavity 16 and a sealed end 15. The cavity's 16 open end is located opposite the sealed end 15. Fig. 2 and 3 show the sleeve 13 in a condition prior forming using pressing. The cavity 16 has such size that the end of the wire bundle 11 which is pulled out of the lead body 5 before that may easily slid into the cavity 16.

Fig. 2 depicts the lead body 5 (without any further details about the accommodation of the wires or wire bundles within the lead body 5) and one wire bundle 11 pulled out of the lead body 5. This is done e.g. by tweezers. Further, the end of the pulled wire bundle 11 extending from the lead body 5 is slid into the cavity 16 of the sleeve 13 until the wire bundle 11 abuts to the inner surface of the sealed end 15 (see Fig. 3). The material of the sleeve 13 is, for example, an implantable metal. Fig. 2 shows the state in which the pulled wire bundle 11 is accommodated within the cavity 16 of the sleeve 13 prior the forming step.

In the next step the sleeve 13 is formed by pressing, such that the thickness of the sleeve is reduced (the sleeve is flattened) thereby reducing the volume of the cavity 16 such that the sleeve 13 displaces any electrically isolating material and establishes an electrical and mechanical connection between the wire strands of the wire bundle 11 and the sleeve 13. Additionally, the wire bundle 11 is sealingly surrounded by the sleeve 13. Formed sleeve 13 is referred to by reference number 13a in the following.

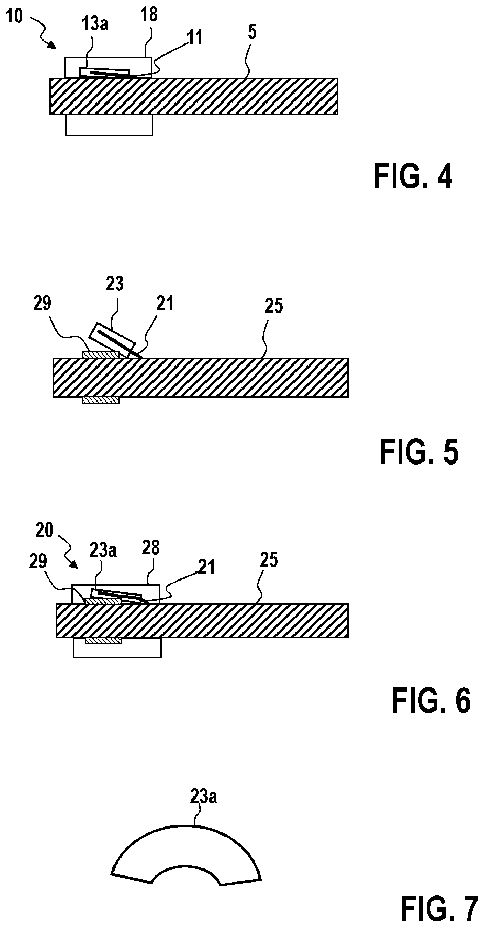

Then, the elongated lead body 5 is inserted into a ring-shaped annular electrode 18 and the annular electrode 18 is moved along the longitudinal direction of the lead body 5 (i.e. the direction of the longitudinal axis 17, see Fig. 2) until it is accommodated over the pressed sleeve 13a and the electrically conducting wire bundle 11. The annular electrode 18 covers the sleeve, the wire bundle 11 (at an end section which is pulled from the lead body 5) as it is shown in Fig. 4. Accordingly, the wire bundle is hermetically sealed by the sleeve 13 having the sealed end 15 and by the annular electrode 18 (see Fig. 4).

In another embodiment shown in Fig. 5 and 6, there is an additional annular element 29 provided. The annular element 29 has an inner diameter which is approximately the outer diameter of the lead body 25. Further, a sleeve 23 and a wire bundle 21 which is pulled out and extends from the lead body 25 is shown.

The sleeve 23 is fixed at the wire bundle 21 end in the same way as in the first embodiment. After forming by pressing the sleeve 23a is clamped between the annular element 29 and the annular electrode 28 that is attached in a similar way as annular electrode 18.

Finally, in Fig. 7 an example of a pressed sleeve 23a is depicted which has an arcuate shape (see top view of Fig. 7) such that it is better adapted to the outer surface of the lead body 25 or the annular element 29 thereby reducing the thickness of the electrode 10.

By the above method and construction of a lead according to the present invention, a hermetic seal for the wire or wire bundle is provided preventing leakage in a cost-effective and automatable way wherein at the same time, the electrical and mechanical connection is highly reliable and durable.

## Claims

1. A method for manufacturing an implantable lead (1) comprising an elongated lead body (5, 25) with a plurality of wires or wire bundles (11, 21) and an electrically isolating outer layer with the following steps:
- Providing a sleeve (13, 23) having a sealed end and an open cavity (16) opposite the sealed end (15) and comprising electrically conducting material;
- Pulling at least one wire or wire bundle (11, 21) out of the lead body (5, 25);
- Positioning the pulled end of the at least one wire or wire bundle (11, 21) within the cavity (16) of the sleeve (13, 23);
- Forming the sleeve (13, 23) using a pressing tool such that the volume of the cavity (16) is reduced, that the wire or wire bundle (11, 21) is fixed within the sleeve (13a, 23a) and that an electrical connection of the at least one wire or wire bundle (11, 21) and the formed sleeve (13a, 23a) is established;
- Attaching a contact element to the lead body (15, 25) such that the contact element fixes the formed sleeve (13a, 23a) at the lead body (5, 25) and establishes an electrical connection to the sleeve (13a, 23a).

2. The method of claim 1, wherein the contact element is an annular electrode (18, 28), wherein the formed sleeve (13a, 23a) is fixed between the annular electrode (18, 28) and said outer layer of the lead body (5, 25).

3. The method of claim 2, wherein an annular element (29) is provided and positioned at the outer layer such that the formed sleeve (13a, 23a) is located between the annular element (29) and the annular electrode (18, 28).

4. The method of any one of the claims 2 to 3, wherein the annular electrode (18, 28) is accommodated such that it fully covers the formed sleeve (13a, 23a) and the length of the annular electrode (18, 28) is greater than the length of the formed sleeve (13a, 23a).

5. The method of any one of the previous claims, wherein the sleeve (23a) is pressed into an arcuate shape.

6. The method of any one of the previous claims, wherein the end of the sleeve (13, 23) is sealed by pressing and/or welding, for example laser welding or resistance welding.

7. The method of any one of the previous claims, wherein the sleeve (13, 23) forming is provided under heating.

8. The method of any one of the previous claims, wherein another electrically isolating layer is applied at the outer surface of the annular electrode (18, 28).

9. An implantable lead (1) comprising an elongated lead body (5, 25) with a plurality of wires or wire bundles (11, 21) and an electrically isolating outer layer, wherein the implantable lead (1) further comprises a formed sleeve (13a, 23a) comprising an electrically conducting material, wherein at least one predetermined wire or wire bundle (11, 21) passes through said outer layer and is positioned and sealed within an inner cavity (16) of the formed sleeve (13a, 23a) so that an electrical connection of the at least one wire or wire bundle (11, 21) and the formed sleeve (13a, 23a) is established via the electrical conducting material, wherein the implantable lead further comprises a contact element that is configured to fix the formed sleeve (13a, 23a) at the lead body (5, 25) and is electrically connected to the contact element.

10. The implantable lead of claim 9 manufactured by the method of anyone of the claims 1 to 8.

11. The implantable lead of claim 9 or 10, wherein the contact element is an annular electrode (18, 28) which covers and fixes the formed sleeve (13a, 23a) at the outer surface of the lead body (5, 25).

12. The implantable lead of claim 11, wherein the length of the annular electrode (18, 28) is greater than the length of the formed sleeve (13a, 23a).

13. The implantable lead of any one of the claims 11 to 12, further comprising an annular element (29), wherein the annular element (29) is located at the outer layer and the formed sleeve (13a, 23a) is located between the annular element (29) and the annular electrode (18, 28), wherein the length of the annular electrode (18, 28) is greater than the length of the annular element (29).

14. The implantable lead of any one of the claims 11 to 13, wherein the annular electrode (18, 28) comprises another electrically isolating layer at its outer surface.

15. The implantable lead of any one of the claims 9 to 14, wherein the at least one wire or wire bundle (11, 21) is accommodated within the lead body (5, 25) in a helical or meandering shape.

## Patentansprüche

1. Verfahren zur Herstellung einer implantierbaren Leitung (1), die einen länglichen Leitungskörper (5, 25) mit einer Vielzahl von Drähten oder Drahtbündeln (11, 21) und einer elektrisch isolierenden äußeren Schicht umfasst, mit den folgenden Schritten:
- Bereitstellen einer Hülse (13, 23), die ein abgedichtetes Ende und einen offenen Hohlraum (16) gegenüber dem abgedichteten Ende (15) aufweist und elektrisch leitendes Material umfasst;
- Herausziehen von mindestens einem Draht oder Drahtbündel (11, 21) aus dem Leitungskörper (5, 25);
- Positionieren des gezogenen Endes des mindestens einen Drahtes oder Drahtbündels (11, 21) innerhalb des Hohlraums (16) der Hülse (13, 23);
- Formen der Hülse (13, 23) mit einem Presswerkzeug, so dass das Volumen des Hohlraums (16) reduziert wird, dass der Draht oder das Drahtbündel (11, 21) innerhalb der Hülse (13a, 23a) fixiert wird und dass eine elektrische Verbindung des mindestens einen Drahtes oder Drahtbündels (11, 21) und der geformten Hülse (13a, 23a) hergestellt wird
- Anbringen eines Kontaktelements an dem Leitungskörper (15, 25), so dass das Kontaktelement die geformte Hülse (13a, 23a) an dem Leitungskörper (5, 25) fixiert und eine elektrische Verbindung zu der Hülse (13a, 23a) herstellt.

2. Das Verfahren nach Anspruch 1, wobei das Kontaktelement eine ringförmige Elektrode (18, 28) ist, wobei die geformte Hülse (13a, 23a) zwischen der ringförmigen Elektrode (18, 28) und der äußeren Schicht des Leitungskörpers (5, 25) fixiert ist.

3. Das Verfahren nach Anspruch 2, wobei ein ringförmiges Element (29) bereitgestellt und an der äußeren Schicht so positioniert ist, dass sich die geformte Hülse (13a, 23a) zwischen dem ringförmigen Element (29) und der ringförmigen Elektrode (18, 28) befindet

4. Das Verfahren nach einem der Ansprüche 2 bis 3, wobei die ringförmige Elektrode (18, 28) so untergebracht wird, dass sie die geformte Hülse (13a, 23a) vollständig bedeckt und die Länge der ringförmige Elektrode (18, 28) größer ist als die Länge der geformten Hülse (13a, 23a).

5. Das Verfahren nach einem der vorhergehenden Ansprüche, wobei die Hülse (23a) in eine bogenförmige Form gepresst wird.

6. Das Verfahren nach einem der vorhergehenden Ansprüche, wobei das Ende der Hülse (13, 23) durch Pressen und/oder Schweißen, z. B. Laserschweißen oder Widerstandsschweißen, abgedichtet wird.

7. Das Verfahren nach einem der vorhergehenden Ansprüche, wobei das Formen der Hülse (13, 23) unter Erwärmung erfolgt.

8. Das Verfahren nach einem der vorhergehenden Ansprüche, wobei eine weitere elektrisch isolierende Schicht auf der Außenfläche der ringförmigen Elektrode (18, 28) aufgebracht wird.

9. Implantierbare Leitung (1), die einen länglichen Leitungskörper (5, 25) mit einer Vielzahl von Drähten oder Drahtbündeln (11, 21) und einer elektrisch isolierenden äußere Schicht umfasst, wobei die implantierbare Leitung (1) ferner eine geformte Hülse (13a, 23a) umfasst, die ein elektrisch leitendes Material umfasst, wobei mindestens ein vorbestimmter Draht oder Drahtbündel (11, 21) durch die äußere Schicht hindurchgeht und in einem inneren Hohlraum (16) der geformten Hülse (13a, 23a) positioniert und versiegelt ist, so dass eine elektrische Verbindung des mindestens einen Drahtes oder Drahtbündels (11, 21) und der geformten Hülse (13a, 23a) über das elektrisch leitende Material hergestellt wird, wobei die implantierbare Leitung ferner ein Kontaktelement umfasst, das so konfiguriert ist, dass es die geformte Hülse (13a, 23a) an dem Leitungskörper (5, 25) fixiert, und das elektrisch mit dem Kontaktelement verbunden ist.

10. Die implantierbare Leitung nach Anspruch 9, hergestellt nach dem Verfahren gemäß einem der Ansprüche 1 bis 8.

11. Die implantierbare Leitung nach Anspruch 9 oder 10, wobei das Kontaktelement eine ringförmige Elektrode (18, 28) ist, die die geformte Hülse (13a, 23a) an der Außenfläche des Leitungskörpers (5, 25) bedeckt und fixiert.

12. Die implantierbare Leitung nach Anspruch 11, wobei die Länge der ringförmigen Elektrode (18, 28) größer ist als die Länge der geformten Hülse (13a, 23a).

13. Die implantierbare Leitung nach einem der Ansprüche 11 bis 12, ferner mit einem ringförmigen Element (29), wobei das ringförmige Element (29) an der äußeren Schicht angeordnet ist und die geformte Hülse (13a, 23a) zwischen dem ringförmigen Element (29) und der ringförmigen Elektrode (18, 28) angeordnet ist, wobei die Länge der ringförmigen Elektrode (18, 28) größer ist als die Länge des ringförmigen Elements (29).

14. Die implantierbare Leitung nach einem der Ansprüche 11 bis 13, wobei die ringförmige Elektrode (18, 28) an ihrer Außenfläche eine weitere elektrisch isolierende Schicht aufweist.

15. Die implantierbare Leitung nach einem der Ansprüche 9 bis 14, wobei der mindestens eine Draht oder das mindestens eine Drahtbündel (11, 21) im Leitungskörper (5, 25) schraubenförmig oder mäanderförmig untergebracht ist.

## Revendications

1. Procédé de fabrication d'un câble implantable (1) comprenant un corps de câble allongé (5, 25) avec une pluralité de fils ou de faisceaux de fils (11, 21) et une couche externe électriquement isolante avec les étapes suivantes :
- fournir un manchon (13, 23) ayant une extrémité scellée et une cavité ouverte (16) opposée à l'extrémité scellée (15) et comprenant un matériau électriquement conducteur ;
- tirer au moins un fil ou faisceau de fils (11, 21) hors du corps de câble (5, 25) ;
- positionner l'extrémité tirée de l'au moins un fil ou faisceau de fils (11, 21) au sein de la cavité (16) du manchon (13, 23) ;
- former le manchon (13, 23) en utilisant un outil de pression de telle sorte que le volume de la cavité (16) est réduit, de telle sorte que le fil ou le faisceau de fils (11, 21) est fixé au sein du manchon (13a, 23a) et de telle sorte qu'une connexion électrique de l'au moins un fil ou faisceau de fils (11, 21) et le manchon (13a, 23a) formé est établie ;
- attacher un élément de contact au corps de câble (15, 25) de telle sorte que l'élément de contact fixe le manchon (13a, 23a) formé au corps de câble (5, 25) et établit une connexion électrique jusqu'au manchon (13a, 23a).

2. Procédé selon la revendication 1, dans lequel l'élément de contact est une électrode annulaire (18, 28), dans lequel le manchon (13a, 23a) formé est fixé entre l'électrode annulaire (18, 28) et ladite couche externe du corps de câble (5, 25).

3. Procédé selon la revendication 2, dans lequel un élément annulaire (29) est pourvu et positionné au niveau de la couche externe de telle sorte que le manchon (13a, 23a) formé est situé entre l'élément annulaire (29) et l'électrode annulaire (18, 28).

4. Procédé selon l'une quelconque des revendications 2 à 3, dans lequel l'électrode annulaire (18, 28) est accueillie de telle sorte qu'elle recouvre entièrement le manchon (13a, 23a) formé et la longueur de l'électrode annulaire (18, 28) est supérieure à la longueur du manchon (13a, 23a) formé.

5. Procédé selon l'une quelconque des revendications précédentes, dans lequel le manchon (23a) est pressé en une forme arquée.

6. Procédé selon l'une quelconque des revendications précédentes, dans lequel l'extrémité du manchon (13, 23) est scellée par pression et/ou soudage, par exemple soudage au laser ou soudage par résistance.

7. Procédé selon l'une quelconque des revendications précédentes, dans lequel l'étape consistant à former le manchon (13, 23) est pourvue sous chauffage.

8. Procédé selon l'une quelconque des revendications précédentes, dans lequel une autre couche électriquement isolante est appliquée au niveau de la surface externe de l'électrode annulaire (18, 28).

9. Câble implantable (1) comprenant un corps de câble (5, 25) allongé avec une pluralité de fils ou de faisceaux de fils (11, 21) et une couche externe électriquement isolante, dans lequel le câble implantable (1) comprend en outre un manchon (13a, 23a) formé comprenant un matériau électriquement conducteur, dans lequel au moins un fil ou faisceau de fils (11, 21) prédéterminé passe à travers ladite couche externe et est positionné et scellé à l'intérieur d'une cavité interne (16) du manchon (13a, 23a) formé de telle sorte qu'une connexion électrique de l'au moins un fil ou faisceau de fils (11, 21) et du manchon (13a, 23a) formé est établie par l'intermédiaire du matériau électriquement conducteur, dans lequel le câble implantable comprend en outre un élément de contact qui est conçu pour fixer le manchon (13a, 23a) formé au niveau du corps de câble (5, 25) et est électriquement connecté à l'élément de contact.

10. Câble implantable selon la revendication 9 fabriqué par le procédé selon l'une quelconque des revendications 1 à 8.

11. Câble implantable selon la revendication 9 ou 10, dans lequel l'élément de contact est une électrode annulaire (18, 28) qui recouvre et fixe le manchon (13a, 23a) formé au niveau de la surface externe du corps de câble (5, 25).

12. Câble implantable selon la revendication 11, dans lequel la longueur de l'électrode annulaire (18, 28) est supérieure à la longueur du manchon (13a, 23a) formé.

13. Câble implantable selon l'une quelconque des revendications 11 à 12, comprenant en outre un élément annulaire (29), dans lequel l'élément annulaire (29) est situé au niveau de la couche externe et le manchon (13a, 23a) formé est situé entre l'élément annulaire (29) et l'électrode annulaire (18, 28), dans lequel la longueur de l'électrode annulaire (18, 28) est supérieure à la longueur de l'élément annulaire (29).

14. Câble implantable selon l'une quelconque des revendications 11 à 13, dans lequel l'électrode annulaire (18, 28) comprend une autre couche électriquement isolante au niveau de sa surface externe.

15. Câble implantable selon l'une quelconque des revendications 9 à 14, dans lequel l'au moins un fil ou faisceau de fils (11, 21) est accueilli à l'intérieur du corps de câble (5, 25) sous une forme hélicoïdale ou sinueuse.
